# EUROPEAN PATENT SPECIFICATION

(11) **EP 2 954 930 B1**
(45) Date of publication and mention of the grant of the patent: **10.04.2019**
(21) Application number: 15170074.7
(22) Date of filing: 01.06.2015
(51) Int. Cl.: A61N 1/375

(54) **DELIVERY SYSTEM FOR AN IMPLANTABLE MEDICAL DEVICE**
ABGABESYSTEM FÜR EINE IMPLANTIERBARE MEDIZINISCHE VORRICHTUNG
SYSTEME DE DISTRIBUTION POUR UN DISPOSITIF MEDICAL IMPLANTABLE

(30) Priority: 10.06.2014 US 201462009935 P
(43) Date of publication of application: 16.12.2015
(73) Proprietor: BIOTRONIK SE & Co. KG, 12359 Berlin (DE)
(72) Inventor: Müssig, Dirk, West Linn, 97068 (US); Stotts, Larry, Tigard, Oregon 97224 (US)
(74) Representative: Galander, Marcus

(56) References cited:
- US-A1- 2006 085 041
- US-A1- 2013 131 693
- US-A1- 2013 253 343

## Description

The invention relates to a delivery system for an implantable medical device in a human or animal body.

The installation of implantable leadless pacemakers demands surgical procedures that employ catheter-based tooling support. Such installations widely differ from the pocket-based patient interfacing associated with the implantation of traditional lead-based pacemakers. For repositioning and especially for explanting an implantable leadless pacemaker a different tool has to be introduced into the body. In many cases it is desirable to introduce the pacemaker through the femoral and not the jugular vasculature which requires a high flexibility of the catheter.

In US 3,835,864 A, a delivery device is described which is used to implant an intra-cardiac stimulator through the jugularis. WO 2012/082755 A1 describes a catheter system for retrieving an implantable leadless cardiac pacemaker from a patient. The catheter is not suitable for implantation of the implant. US 2013/0253343 A1 represents the closest prior art with respect to the present invention.

The fixation mechanism of an implantable medical device such as an implantable leadless pacemaker needs to be protected during implantation. This is achieved by using a tube (straw) like structure as a protector cup that is placed around the implantable leadless pacemaker. This tube is longer than the implantable leadless pacemaker so it extends past the fixation mechanism, such as a screw. The distal part of the tube has a soft edge to avoid damage of myocardial tissue due to applied pressure when placing the implantable leadless pacemaker. After the initial fixation of the implantable leadless pacemaker to the myocardium the protective tube needs to be removed to allow the implantable leadless pacemaker to move freely with the movement of the heart. The removal of the tube is achieved by sliding it back towards the atrium. During this phase the implantable leadless pacemaker is still connected to the delivery system via a highly flexible tether. In order to assess correct placement and anchoring of the implantable leadless pacemaker it must not interfere with any structure of the delivery system.

The combined length of the implantable leadless pacemaker implant and the protector cup is longer than the long axis of the heart. That makes it very difficult to achieve a tether mode in which the implantable leadless pacemaker can freely, undisturbed by elements of the delivery system (e.g. the protector cup), move with the myocardium, and to fully release the implantable leadless pacemaker after it is fixated into the myocardium. The required length, i.e. the combined length of the implantable leadless pacemaker and the protective tube, is longer than the distance from the apex of the right ventricle to the distal end of deflected outer sheath of the delivery device which is required to direct the implantable leadless pacemaker from a cranial direction (coming from the femoral vein) to the right ventricle.

One option is to make the implantable leadless pacemaker shorter so that the total length of the implantable leadless pacemaker and protector cup is shorter than the available working length of the long axis of the human heart. However, this solution requires increasing the diameter of the implantable leadless pacemaker because a minimum volume of the pacemaker is required for battery and electronics, and therefore has a negative impact on the implantability due to human vessel diameter.

Another option is to move the protector cup to the outer sheath of the delivery device to allow it being retracted back into the inferior vena cava (IVC). It has the drawback that the outer sheath of the delivery device cannot be steerable and therefore does not allow the precise placement of the implantable leadless pacemaker into the target region.

It is an object of the invention to provide a delivery system for an implantable medical device that allows a more precise placement and release of the implantable medical device. Another object is to provide a system comprising a delivery system and an implantable medical device where delivery, and recapture or explant of the medical device can be performed.

The objects are achieved by the features of the independent claim. The other claims, the description and the drawings disclose favorable embodiments of the invention.

A delivery system for an implantable medical device to be introduced into a human or animal body is proposed, in particular an implantable leadless pacemaker, having an outer sheath and an inner sheath, wherein the inner sheath is configured to be retractable into a lumen of the outer sheath, wherein a distal side of the inner sheath is configured to provide regions which are non-collapsible and reversibly collapsible in diameter, wherein a non-collapsible region is arranged at the distal end of the inner sheath.

The presented invention allows a more precise placement and release of the implantable medical device. Also it is designed to simplify recapturing of a chronically implanted implantable leadless pacemaker during an exchange procedure due to e.g. battery depletion. The region of collapsible diameter with the non-collapsible front end favorably acts as protector cup for the medical device.

Besides a precise placement of the medical device in the target area of the tissue in an animal or human body, a tether mode is enabled without pulling on the medical device, as well as a controlled release of the medical device. Further, more space is provided to allow an easier recapture procedure at battery depletion.

In a tether mode the implantable medical device is released from the intimate connection to the distal delivery tool tip during the primary engagement mode of operation but still connected via a wire or other connection elements in order to test the location and functions of the medical implant before completely releasing it from the delivery tool.

Advantageously, the invention allows moving the protective inner sheath out of the right ventricle and the lower part of the right atrium without changing placement and direction of the outer sheath as well as the position of the medical device. Advantageously, the fixation mechanism of the medical device, such as an implantable leadless pacemaker, can be protected during implantation. Because the inner sheath has a collapsible region at its distal side, it can safely be retracted into the lumen of the outer sheath by reducing its diameter and be pushed out of the outer sheath while expanding its diameter so that it can cover the medical device and the fixation mechanism at the distal end of the medical device, forming a protector cup for the device.

It is not necessary to make the implantable medical device shorter so that the total length (implantable medical device and protector cup) is shorter than the available working length of the long axis of the human or animal heart. Therefore, it is not required to increase the diameter of the medical device for giving a minimum volume of the medical device being required for battery and electronic components, and therefore has no negative impact on the implantability due to human or animal vessel diameter.

It can also be avoided to move the protector cup to the outer sheath of the implant tool to allow it being retracted back into the inferior vena cava (IVC). Therefore, the drawback can be avoided that the outer sheath of the delivery tool cannot be steerable and therefore does not allow the precise placement of the medical device into the target region.

The inventive solution is to design the protective inner sheath in a way that allows it to collapse and to be pulled back into the lumen of the outer sheath. The proposed solution also allows the protective sheath to unfold back into its original shape when pushed back out of the lumen of the outer sheath. This is advantageous for repositioning procedures, in case the interface parameters (e.g. pacing threshold, sensed signal amplitude) are not acceptable for a permanent placement of the medical device or the fixation does not appear to be stable enough to fully release the medical device from a tether mode. In addition this delivery tool is able to support recapturing the medical device for replacement procedures when the device battery is depleted.

According to an advantageous embodiment of the delivery device, the non-collapsible region may comprise a soft material configured to protect tissue while applying pressure when placing the implantable medical device. The soft distal end of the protector cup is not collapsible in diameter, but always remains in the retracted position on the outside of the outer sheath, close to the distal end of it.

According to an advantageous embodiment, the collapsible region may comprise a metal structure having a memory effect, wherein the metal structure may elongate and reduce its diameter by applying a pulling force and which may unfold the metal structure into its non-collapsed form when pushed out of the inner sheath. In particular the metal structure may comprise Nitinol.

In an alternative embodiment, the collapsible region may comprise longitudinal sections of differing thicknesses and/or durability. In particular, in circumferential direction of the inner sheath sections of thicker material may be spaced apart by sections of thinner material.

Advantageously, the sections may comprise polymer material. By constructing the protector cup from polymers of different thicknesses and durability, small thin longitudinal sections of the protector cup can be made of the thicker material to allow for form stability while they are connected with thinner material that folds in when applying transversal force. Also it is conceivable that the medical device itself acts as form to bring the protector cup back in its unfolded stage.

According to another aspect of the invention, a system comprising a delivery tool and a medical implant is proposed, the medical device having a distal end and an opposing proximal end with a fastener provided for interaction with a coupling element of the delivery system, the delivery system comprising an outer sheath and an inner sheath, wherein the inner sheath is configured to be retractable into a lumen of the outer sheath, and the inner sheath being arranged to cover temporarily at least a proximal end of the implantable medical device, wherein a distal end of the inner sheath is configured to provide regions non-collapsible and reversibly collapsible in diameter.

According to an advantageous embodiment of the system, the non-collapsible region may comprise a soft material configured to protect tissue while applying pressure when placing the implantable medical device. The soft distal end of the protector cup is not collapsible in diameter, but always remains in the retracted position on the outside of the outer sheath, close to the distal end of it.

According to an advantageous embodiment of the system, the collapsible region may comprise a metal structure having a memory effect, wherein the metal structure elongates and reduces its diameter by applying a pulling force and which unfolds the metal structure into its non-collapsed form when pushed out of the inner sheath.

According to an alternative embodiment of the system, the collapsible region may comprise longitudinal sections of differing thicknesses and/or durability.

The present invention together with the above-mentioned and other objects and advantages may best be understood from the following detailed description of the embodiments, but not restricted to the embodiments, wherein is shown in:
- Fig. 1: a system comprising a delivery system and a implantable medical device having an inner sheath providing a collapsible metal structure;
- Fig. 2: the distal side of the delivery system of Figure 1 showing a first embodiment of the inner sheath;
- Fig. 3: a second embodiment of an inner sheath providing sections of different thicknesses and durability in its unfolded state outside the outer sheath and in its collapsed state inside the outer sheath;
- Fig. 4: a system comprising a delivery tool and an implantable leadless pacemaker with the implantable leadless pacemaker attached to the heart but still connected to the delivery tool;
- Fig. 5: a detail of a distal region of an inner sheath with collapsible and non-collapsible regions.

In the drawings, like elements are referred to with equal reference numerals. The drawings are merely schematic representations, not intended to portray specific parameters of the invention. Moreover, the drawings are intended to depict only typical embodiments of the invention and therefore should not be considered as limiting the scope of the invention.

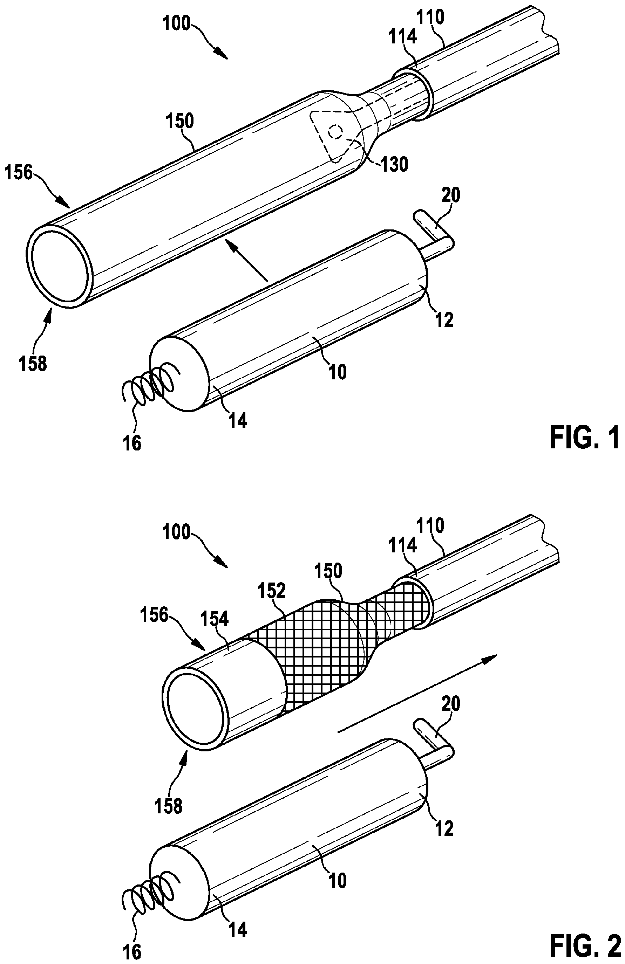

Figure 1 depicts a part of a system comprising a delivery tool 100, such as a catheter, for an implantable medical device 10 (depicted lying beside the delivery tool 100) to be introduced into a human or animal body. The medical device 10 may be an implantable leadless pacemaker, having a distal end 14 and an opposing proximal end 12 with a fastener 20 provided for interaction with a coupling element 130 of the delivery tool 100.

The implantable medical device 10 has a distal end 14 and a proximal end 12. The distal end 14 can be attached to the surrounding tissue (not shown) by means of a fixation mechanism 16 such as a helix or the like which can be screwed into the tissue (not shown) by rotating the implantable medical device 10. The delivery tool 100 is provided to advance the implantable medical device 10 and to rotate the implantable medical device 10. In order to transmit torque to the implantable medical device 10, the implantable medical device 10 is intimately attached to a coupling element 130 at the distal tip of the delivery tool via a fastener 20 arranged at the proximal end 12 of the implantable medical device 10. In this embodiment, the fastener 20 is a modified flat fin, but may be a ball, a flexible neck or the like. For the fin as fastener 20 the coupling element 130 may be a gripper or a cup protruding from a metal tip.

The delivery tool 100 comprises an outer sheath 110 and an inner sheath 150, wherein the inner sheath 150 is configured to be retractable into a lumen of the outer sheath 110. The distal region of inner sheath 150 is arranged to cover temporarily the implantable medical device 10 acting as protector cup during the implant procedure. A distal end 156 of the inner sheath 150 is configured to provide collapsible and non-collapsible regions 152, 154, forming the protector cup. The coupling element 130 is attached to a torque transmitting tube or wire inside the inner sheath 150.

The non-collapsible region 154 comprises a soft material configured to protect tissue while applying pressure when placing the implantable medical device 10. The collapsible region 154 provides varying diameters of the distal side 156 of the inner sheath 150 inside and outside the outer sheath 110.

Figure 2 illustrates the distal side of the delivery system of Figure 1 showing a first embodiment of the inner sheath 150. Again, the implantable medical device 10 is shown lying beside the delivery device 100.

The inner sheath 150 has a soft tip 154 at its distal part 156 which is not collapsible in diameter. A region 152 having a collapsible diameter is arranged at the proximal part of the soft tip and comprises a mesh-like structure which can be moved into the outer sheath 110. The mesh-like structure of the collapsible region 152 may be composed of e.g. Nitinol. The collapsible region 152 comprises a metal structure having a memory effect, such that the metal structure elongates and reduces its diameter by applying a pulling force and which unfolds the metal structure into its non-collapsed form when pushed out of the inner sheath 150.

Because of the two regions 152, 154 of the protector cup, i.e. the distal non-collapsible soft tip and the proximal collapsible structure, when the inner sheath is pulled back into the outer sheath 110, the collapsible region 152 collapses in diameter and can be moved into the lumen of the outer sheath 110 (indicated by an arrow in Figure 2). When pushed out again the collapsible region 152 unfolds back into its original shape and forms a protective element around the implantable medical device 10.

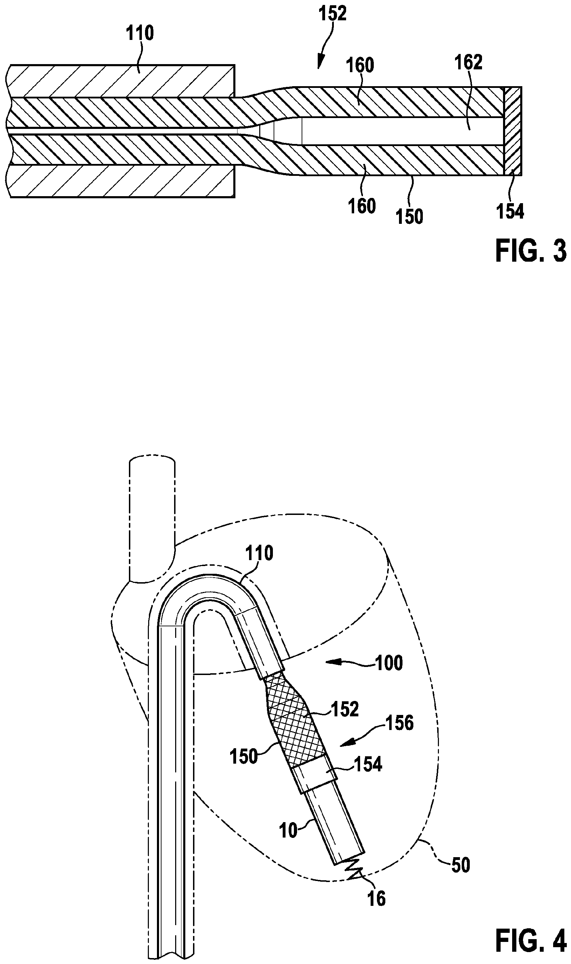

Figure 3 illustrates a second embodiment of an inner sheath 150 forming a protector cup providing sections of different thicknesses and durability in its unfolded state outside the outer sheath 110 and in its collapsed state inside the outer sheath 110. The collapsible region 152 comprises longitudinal sections 160, 162 of differing thicknesses and/or durability. The thicker sections 160 provide for form stability of the distal side 156 of the inner sheath 150 and are connected to each other by sections 162 of thinner material.

The protector cup may be composed of one or more polymers. The small thin longitudinal sections of the protector cup can be made of the thicker material 160 to allow for form stability while they are connected with thinner material 162 that folds in when applying transversal force. The implantable medical device 10 (Figures 1, 2) itself may act as form to bring the protector cup back in its unfolded stage outside the outer sheath 110.

Figure 4 shows a system comprising a delivery tool 100 and an implantable leadless pacemaker as implantable medical device 10 during implant into a human or animal heart 50, with the implantable leadless pacemaker attached to the heart 50 but still connected to the delivery tool 100.

The fixation mechanism 16 of the implantable leadless pacemaker is protected during implantation. This is achieved using the inner sheath 150 that is placed around the implantable leadless pacemaker. The distal part 158 of the inner sheath 150 has a soft edge 154 to avoid damage of myocardial tissue due to applied pressure when placing the implantable leadless pacemaker. After the initial fixation of the implantable leadless pacemaker to the myocardium the inner sheath 150 has to be removed to allow the implantable leadless pacemaker to move freely with the movement of the heart 50. The removal of the inner sheath 150 is achieved by sliding it back towards the atrium by moving it into the lumen of the outer sheath 100. During this phase the implantable medical device 10 is still connected to the delivery system via a highly flexible tether. According to the inventive delivery tool 100, the implantable medical device 10 does not interfere with any structure of the delivery tool 1 00 while assessing correct placement and anchoring of the implantable medical device 10.

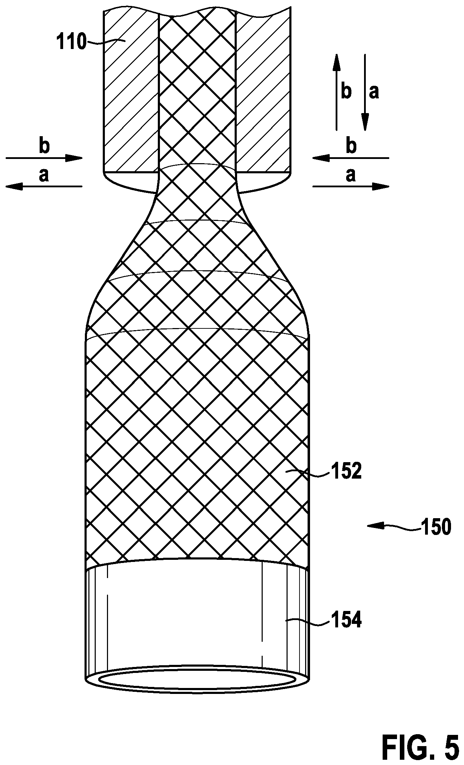

Figure 5 depicts the change in diameter of the collapsible region 152 of the distal region 156 of the inner sheath 150 with collapsible and non-collapsible regions 152, 154. Arrows labelled with letter "b" indicate how the diameter of the inner sheath 150 collapses in the region 152 when the inner sheath is moved back into the lumen of the outer sheath 110.

The inventive delivery tool 100 also allows the protective inner sheath 150 to unfold back into its original shape when pushed back out of the lumen of the outer sheath 110. This is indicated by arrows labelled with letter "a". This is essential for repositioning procedures, in case the interface parameters (e.g. pacing threshold, sensed signal amplitude) are not acceptable for a permanent placement of the implantable medical device 10 or the fixation does not appear to be stable enough to fully release the implantable leadless pacemaker from the tether mode. In addition this mechanism the delivery tool 100 supports recapturing the implantable leadless pacemaker for replacement procedures when the device battery is depleted.

## Claims

1. A system comprising a delivery tool (100) for an implantable medical device (10) to be introduced into a human or animal body and an implantable medical device (10), the implantable medical device (10) having a distal end (14) and an opposing proximal end (12) with a fastener (20) provided for interaction with a coupling element (130) of the delivery tool (100), the delivery tool (100) comprising an outer sheath (110) and an inner sheath (150), wherein the inner sheath (150) is configured to be retractable into a lumen of the outer sheath (110), the inner sheath (150) being arranged to cover temporarily at least a proximal end (12) of the implantable medical device (10), wherein a distal end of the inner sheath (150) is configured to provide a region (152), which is reversibly collapsible in diameter, and a region (154), which is non-collapsible in diameter,
**characterized in, that** the non-collapsible region (154) is arranged at the distal end (158) of the inner sheath (150).

2. The system according to claim 1, **characterized in, that** inner sheath (150) being arranged to cover temporarily the implantable medical device (10) acting as protector cup during the implant procedure.

3. The system according to claim 1 or 2, **characterized in, that** the collapsible region (152) comprises a metal structure having a memory effect, wherein the metal structure elongates and reduces its diameter by applying a pulling force and which unfolds the metal structure into its non-collapsed form when pushed out of the outer sheath (110).

4. The system according to claim 3, **characterized in, that** the metal structure comprises Nitinol.

5. The system according to claim 1 or 2, **characterized in, that** the collapsible region (152) comprises longitudinal sections (160, 162) of differing thicknesses and/or durability.

6. The system according to claim 5, wherein in circumferential direction of the inner sheath (150) sections (160) of thicker material are spaced apart by sections (162) of thinner material.

7. The system according to claim 5 or 6, wherein the sections (160, 162) comprise polymer material.

8. The system according to claim 1 or 2, **characterized in, that** the implantable medical device (10) is a leadless pacemaker.

9. The system according to claim 8, **characterized in, that** the inner sheath (150) is configured to be temporarily placed around the implantable leadless pacemaker.

## Patentansprüche

1. System, das ein Abgabeinstrument (100) für eine in einen menschlichen oder tierischen Körper einzuführende implantierbare medizinische Vorrichtung (10) und eine implantierbare medizinische Vorrichtung (10) aufweist,
wobei die implantierbare medizinische Vorrichtung (10) ein distales Ende (14) und ein entgegengesetztes proximales Ende (12) mit einem Befestigungselement (20) hat, das zum Zusammenwirken mit einem Kopplungselement (130) des Abgabeinstruments (100) vorgesehen ist, wobei das Abgabeinstrument (100) eine Außenhülle (110) und eine Innenhülle (150) aufweist, wobei die Innenhülle (150) so gestaltet ist, dass sie in ein Lumen der Außenhülle (110) einziehbar ist, wobei die Innenhülle (150) angeordnet ist, um zeitweise zumindest ein proximales Ende (12) der implantierbaren medizinischen Vorrichtung (10) zu bedecken, wobei ein distales Ende der Innenhülle (150) so gestaltet ist, dass es eine Region (152), die umkehrbar im Durchmesser zusammendrückbar ist, und eine Region (154), die im Durchmesser nicht zusammendrückbar ist, bereitstellt,
**dadurch gekennzeichnet, dass** die nicht zusammendrückbare Region (154) am distalen Ende (158) der Innenhülle (150) angeordnet ist.

2. System nach Anspruch 1, **dadurch gekennzeichnet, dass** die Innenhülle (150) zum zeitweisen Bedecken der implantierbaren medizinischen Vorrichtung (10) angeordnet ist, wobei sie während des Implantationsvorgangs als Schutzkelch wirkt.

3. System nach Anspruch 1 oder 2, **dadurch gekennzeichnet, dass** die zusammendrückbare Region (152) ein Metallgebilde mit einem Gedächtniseffekt aufweist, wobei das Metallgebilde sich durch Anwenden einer Ziehkraft verlängert und seinen Durchmesser reduziert und der das Metallgebilde in seine nicht zusammengedrückte Form entfaltet, wenn es aus der Außenhülle (110) herausgeschoben wird.

4. System nach Anspruch 3, **dadurch gekennzeichnet, dass** das Metallgebilde Nitinol aufweist.

5. System nach Anspruch 1 oder 2, **dadurch gekennzeichnet, dass** die zusammendrückbare Region (152) langgestreckte Abschnitte (160, 162) verschiedener Dicken und/oder Haltbarkeit aufweist.

6. System nach Anspruch 5, wobei in Umfangsrichtung der Innenhülle (150) Abschnitte (160) aus dickerem Material durch Abschnitte (162) aus dünnerem Material voneinander beabstandet sind.

7. System nach Anspruch 5 oder 6, wobei die Abschnitte (160, 162) Polymermaterial aufweisen.

8. System nach Anspruch 1 oder 2, **dadurch gekennzeichnet, dass** die implantierbare medizinische Vorrichtung (10) ein leitungsloser Schrittmacher ist.

9. System nach Anspruch 8, **dadurch gekennzeichnet, dass** die Innenhülle (150) gestaltet ist, um zeitweise um den implantierbaren leitungslosen Schrittmacher angelegt zu werden.

## Revendications

1. Système comprenant un outil de pose (100) pour un dispositif médical implantable (10) devant être introduit dans un corps humain ou animal, et un dispositif médical implantable (10),
le dispositif médical implantable (10) ayant une extrémité distale (14) et une extrémité proximale (12) opposée avec un système de fixation (20) prévu pour une interaction avec un élément de couplage (130) de l'outil de pose (100), l'outil de pose (100) comprenant une gaine extérieure (110) et une gaine intérieure (150), où la gaine intérieure (150) est configurée pour être rétractable dans une lumière de la gaine extérieure (110), la gaine intérieure (150) étant agencée pour recouvrir temporairement au moins une extrémité proximale (12) du dispositif médical implantable (10), où une extrémité distale de la gaine intérieure -150) est configurée pour fournir une région (152) qui peut se réduire en diamètre de manière réversible et une région (154) qui ne peut pas se réduire en diamètre,
**caractérisé en ce que** la région qui ne peut pas se réduire (154) est agencée à l'extrémité distale (158) de la gaine intérieure (150).

2. Système selon la revendication 1, **caractérisé en ce que** la gaine intérieure (150) étant agencée pour recouvrir temporairement le dispositif médical implantable (10) joue le rôle d'un capuchon protecteur pendant la procédure d'implantation.

3. Système selon la revendication 1 ou la revendication 2, **caractérisé en ce que** la région (152) qui peut se réduire comprend une structure métallique ayant un effet de mémoire, où la structure métallique élargit et réduit son diamètre en appliquant une force de poussée et qui déplie la structure métallique dans sa forme non réduite lorsqu'elle est poussée hors de la gaine extérieure (110).

4. Système selon la revendication 3, **caractérisé en ce que** la structure métallique comprend du nitinol.

5. Système selon la revendication 1 ou la revendication 2, **caractérisé en ce que** la région (152) susceptible de se réduire comprend des sections longitudinales (160, 162) de différentes épaisseurs et/ou durabilités.

6. Système selon la revendication 5, dans lequel, dans la direction circonférentielle de la gaine intérieure (150), des sections (160) de matière plus épaisses sont espacées séparées par des sections (162) de matière plus minces.

7. Système selon la revendication 5 ou la revendication 6, dans lequel les sections (160, 162) comprennent de la matière polymère.

8. Système selon la revendication 1 ou la revendication 2, **caractérisé en ce que** le dispositif médical (10) est un pacemaker sans sonde.

9. Système selon la revendication 8, **caractérisé en ce que** la gaine intérieure (150) est configurée pour être temporairement placée autour du pacemaker sans sonde implantable.
